# EUROPEAN PATENT SPECIFICATION

(11) **EP 3 439 634 B1**
(45) Date of publication and mention of the grant of the patent: **15.07.2020**
(21) Application number: 17718748.1
(22) Date of filing: 28.03.2017
(51) Int. Cl.: A61K 9/00, A61K 47/32, A61K 47/38, A61K 9/70, A61M 35/00, A61K 31/60

(54) **PRODUCT FOR THE TREATMENT OF SKIN LESIONS AND WARTS AND DEVICE FOR THE APPLICATION OF SAID PRODUCT**
PRODUKT ZUR BEHANDLUNG VON HAUTVERLETZUNGEN UND WARZEN SOWIE VORRICHTUNG ZUR ANWENDUNG DES BESAGTEN PRODUKTS
PRODUIT POUR LE TRAITEMENT DE LÉSIONS CUTANÉES ET DE VERRUES, ET DISPOSITIF POUR L'APPLICATION DUDIT PRODUIT

(30) Priority: 05.04.2016 IT UA20162327
(43) Date of publication of application: 13.02.2019
(73) Proprietor: Sixtem Life S.r.l., 59100 Prato (PO) (IT)
(72) Inventor: OTTANELLI, Luciano, 59100 Prato (PO) (IT)
(74) Representative: Vinci, Marcello
(86) International application number: PCT/IB2017/051756
(87) International publication number: WO 2017/175094

(56) References cited:
- WO-A1-2014/042604
- WO-A1-2014/140475
- WO-A1-2015/150705
- WO-A2-2013/131969
- KR-A- 20110 061 085
- US-A- 5 433 950
- US-A1- 2011 086 109
- Anonymous: "New polymer for transparent sun protection", Internet citation, 27 March 2015 (2015-03-27), XP002764636, Retrieved from the Internet: URL:http://www.press.bayer.com/baynews/bay news.nsf/id/New-polymer-for-transparent-su n-protection [retrieved on 2016-11-22]

## Description

The present patent concerns the devices and products for the treatment of skin lesions and warts, and in particular it concerns a new salicylic acid-based product for the treatment of skin lesions and warts and an applicator device for said product.

The currently known processes for self-medication in the treatment of skin lesions and warts are substantially two, that is, cryotherapy and the application of salicylic acid-based solutions.

Cryotherapy is a therapeutic process using fluids at extremely low temperatures for the localized treatment of skin lesions, such as warts or other more or less severe skin diseases.

The controlled and punctual application of the coolant on the lesion causes the freezing of intracellular water and the alteration of proteins and enzymes, producing, within the therapeutic application time frames and the successive thawing, a dermal-epidermal detachment without damaging the derma.

Cryotherapy is presently considered the most effective treatment, as it is characterized by quicker application times, even if at a rather high cost.

Treatments with salicylic acid-based solutions are more widely used. In addition to acting as an antiseptic, salicylic acid also has a keratolytic effect, intended to reduce the mutual adhesion of corneocytes, which are the cells forming the protective layer of the epidermis, destroying the wart through progressive desquamation and activating cellular renewal.

Furthermore, the acidity of the solution creates an environment which is hostile to the virus and causes its extinction.

The treatment with salicylic acid-based solutions is more economic but requires more applications and therefore takes longer to be effective.

US2011/0086109 A1 discloses a low ether gel composition for application to skin comprising salicylic acid, nitrocellulose, and volatile ingredients.

Most of the salicylic acid-based solutions appear as substantially liquid solutions and are difficult to apply correctly and in a localized manner on the lesion, without affecting the surrounding skin.

This treatment, in fact, generally affects also the areas around the lesion, thus damaging them.

These solutions are generally applied by means of a small brush, which requires a certain amount of manual skill, in particular in the case where the part to be treated is difficult to reach.

The salicylic acid-based solution contains also ether and other aggressive components which, in addition to being stinking, also make the treatment extremely troublesome.

Once the solution has been spread on the lesion, it is necessary to apply a plaster on the treated part, in order to allow the solution to work and also avoid contagion.

In order to overcome all the drawbacks mentioned above, a new type of product has been designed and developed, which is a salicylic acid-based product for the treatment of skin lesions and warts, together with an applicator device for the application of the new product.

The main object of the present invention is to resolve all the drawbacks posed by the traditional salicylic acid-based products.

In particular, it is an important object of the invention to provide a new product that can be applied more precisely and easily thanks to its gel formulation, which prevents it from leaking on the surrounding areas.

It is another object of the present invention to provide a product that creates a film on the lesion or wart and releases the active substances gradually, also forming a protective barrier.

The present invention relates to a salicylic acid-based product for the use in the treatment of skin lesions and warts, characterized in that it comprises:
- salicylic acid;
- a polymer that acts as a film former and is suited to combine with the substances contained in the new product so as to create, once the product has been applied to the area to be treated, a water-repellent film suited to retain the substance and release it gradually, wherein said polymer acting as a film former is a polyurethane;
- lactic acid; and
- Melaleuca Alternifolia Leaf Oil.

The invention also relates to an applicator device containing said composition, as defined in claim 6.

An important advantage offered by the present invention is represented by the fact that it reduces the number of applications required, thanks to said gradual and prolonged release of the active substances.

An important advantage offered by the present invention lies in that it does not require the application of plasters once the product has been spread, since said protective barrier is resistant to water.

Another important advantage offered by the present invention lies in that it limits contagion and the spread of infections, thanks to the presence of said film.

It is another object of the present invention to provide a product that makes the treated area immediately visible and easy to identify, thanks to the colouring agent contained in the product.

Another advantage offered by the present invention is constituted by the fact that its formulation does not contain foul-smelling ingredients, thus making the use of the new product more pleasant.

These and other direct and complementary objects are achieved by the new salicylic acid-based product for the treatment of skin lesions and warts and by the applicator device suitable for the application of the new product.

The new product comprises salicylic acid and a polymer that acts as a film former and is suited to combine with the substances contained in the new product so that, once the product has been applied to the area to be treated, it creates a water-repellent film suited to hold the substance and release it gradually.

In particular, said polymer acting as a film former is a polyurethane, for example of the type currently known under the name of "Bayer© Bacusan C 2000".

Said polyurethane combines with the substances contained in the new product, thus creating a matrix which holds the ingredients.

The product is in the form of gel and, once applied, in just a few minutes it creates a water-repellent film which not only releases the active substances gradually, but also protects the lesion and prevents it from spreading, while at the same time avoiding contagion.

The new product comprises also lactic acid, which is suited to develop a synergy with said salicylic acid, reinforcing the keratolytic action and also acting as a disinfectant.

The new product may also comprise hydroxypropylcellulose, for example known as Klucel©, which combines the solubility of solvents, thermoplasticity and surface activity with the thickening and stabilizing properties of the other polymers and makes it easier to obtain the gel formulation.

Once applied, the new product does not require the application of plasters on the treated area, which can also come into contact with water without the product being washed away or dispersed.

The new product may also contain at least one colouring agent, suited to impart a clearly visible colour to the product, for example and preferably light blue, so that the treated area can be clearly identified.

The new product comprises also Melaleuca Alternifolia Leaf Oil, also known as Tea Tree Oil, which imparts a pleasant smell to the product, in addition to having antimicrobial, antiviral and immune-stimulating properties.

The new product may also comprise limonene, which is suited to impart a pleasant smell to the product.

The new product in the form of gel can be conveniently applied by means of an applicator device of the type shaped as a pen, that is, of the type comprising a tubular body intended to contain the new product, an applicator brush or tip fitted in one end of said tubular body, a piston system fitted in the opposite end of said tubular body, and a cap suited to close said tip.

The applicator device makes it possible to apply the product with precision, without damaging the surrounding areas.

The characteristics of the applicator device for the application of the new product are highlighted in greater detail in the following description, with reference to the drawing which is attached hereto by way of non-limiting example.

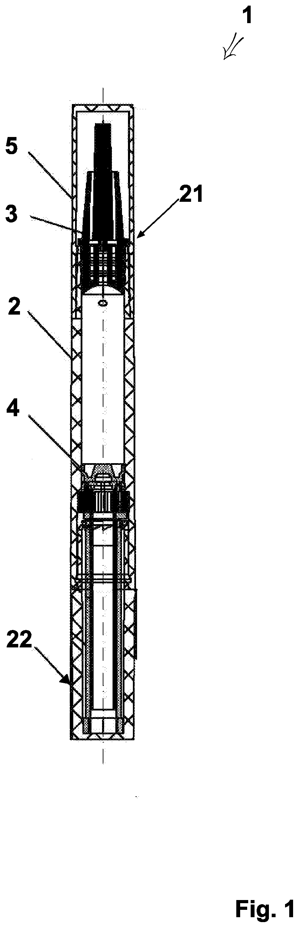

Figure 1 shows a sectional view of an applicator device of the type shaped as a pen (1), which according to a possible embodiment can be used to apply the new product. In particular, said applicator device (1) comprises a tubular body (2) intended to contain the new product, a special applicator tip (3) fitted in one end (21) of said tubular body (2), a dispensing system, for example a piston system (4) fitted in the opposite end (22) of said tubular body (2), and a cap (5) suited to close said tip (3).

In the preferred embodiment, said applicator tip (3) is microholed and has the shape of a flute mouth, which is useful to facilitate a more precise application of the gel.

Said tip (3), furthermore, is conveniently provided with a closing system which prevents the product from leaking out at the end of the dispensing operation, furthermore guaranteeing that said tubular body (2) is tightly closed, in such a way that the product contained therein is correctly preserved and does not deteriorate when it comes in contact with the air.

These are the schematic outlines which are sufficient for the expert in the art to carry out the invention, consequently upon implementation variants may be developed which do not affect the substance of the innovative concepts introduced herein.

Therefore, with reference to the above description and the attached drawing, the following claims are expressed.

## Claims

1. Salicylic acid-based product for the use in the treatment of skin lesions and warts, **characterized in that** it comprises:
- salicylic acid;
- a polymer that acts as a film former and is suited to combine with the substances contained in the new product so as to create, once the product has been applied to the area to be treated, a water-repellent film suited to retain the substance and release it gradually, wherein said polymer acting as a film former is a polyurethane;
- lactic acid;
- Melaleuca Alternifolia Leaf Oil.

2. Product according to claim 1, **characterized in that** it is in the form of gel.

3. Product according to any of the preceding claims, **characterized in that** it also comprises hydroxypropyl cellulose.

4. Product according to any of the preceding claims, **characterized in that** it also comprises at least one colouring agent suited to highlight the applied product with respect to the colour of the skin.

5. Product according to any of the preceding claims, **characterized in that** it also comprises limonene.

6. Applicator device (1) for the application of the product for the treatment of skin lesions and warts according to one or more of the preceding claims, **characterized in that** it is of the type in the shape of a pen, that is, of the type comprising a tubular body (2) containing said product, an applicator tip (3) fitted in one end (21) of said tubular body (2), a dispensing system (4) fitted in the opposite end (22) of said tubular body (2).

## Patentansprüche

1. Auf Salicylsäure basierendes Produkt für die Anwendung zur Behandlung von Hautläsionen und Warzen, **dadurch gekennzeichnet, dass** es Folgendes umfasst:
- Salicylsäure;
- einen Polymer, der als Filmbildner agiert und geeignet ist, sich mit den in dem neuen Produkt enthaltenen Stoffen zu verbinden, so dass nach dem Auftrag des Produkts auf den zu behandelnden Bereich ein wasserabweisender Film entsteht, der geeignet ist, den Stoff zurückzuhalten und graduell abzugeben, wobei das besagte, als Filmbildner agierende Polymer ein Polyurethan ist;
- Milchsäure;
- Teebaumöl.

2. Produkt nach Patentanspruch 1, **dadurch gekennzeichnet, dass** es gelförmig ist.

3. Produkt nach einem jeden der vorstehenden Patentansprüche, **dadurch gekennzeichnet, dass** es auch Hydroxypropylcellulose umfasst.

4. Produkt nach einem jeden der vorstehenden Patentansprüche, **dadurch gekennzeichnet, dass** es außerdem wenigstens ein Färbemittel umfasst, das geeignet ist, das aufgetragene Produkt von der Hautfarbe abzuheben.

5. Produkt nach einem jeden der vorstehenden Patentansprüche, **dadurch gekennzeichnet, dass** es auch Limonen umfasst.

6. Applikatorvorrichtung (1) zum Auftragen des Produkts zur Behandlung von Hautläsionen und Warzen nach einem oder mehreren der vorstehenden Patentansprüche, **dadurch gekennzeichnet, dass** es von stiftförmigem Typ ist, das heißt des Typs, der einen röhrenförmigen Körper (2) umfasst, der das besagte Produkt enthält, sowie eine in ein Ende (21) des besagten röhrenförmigen Körpers (2) eingesetzte Applikatorspitze (3) und ein in das entgegengesetzte Ende (22) des besagten röhrenförmigen Körpers (2) eingesetztes Ausgabesystem (4).

## Revendications

1. Produit à base d'acide salicylique pour l'emploi dans le traitement de lésions cutanées et verrues, **caractérisé en ce qu'il** comprend :
- de l'acide salicylique ;
- un polymère qui agit en tant que filmogène et est apte à se combiner avec les substances contenues dans le nouveau produit de manière à créer, une fois que le produit a été appliqué sur la zone à traiter, une pellicule hydrofuge indiqué pour retenir la substance et la relâcher graduellement, où ledit polymère agissant en tant que filmogène est un polyuréthane ;
- de l'acide lactique ;
- huile de feuilles de Melaleuca Alternifolia.

2. Produit selon la revendication 1, **caractérisé en ce qu'**il est réalisé sous forme de gel.

3. Produit selon l'une quelconque des revendications précédentes, **caractérisé en ce qu'**il comprend en outre de l'hydroxypropylcellulose.

4. Produit selon l'une quelconque des revendications précédentes, **caractérisé en ce qu'**il comprend également au moins un agent colorant indiqué pour mettre en évidence le produit appliqué par rapport à la couleur de la peau.

5. Produit selon l'une quelconque des revendications précédentes, **caractérisé en ce qu'**il comprend en outre du limonène.

6. Dispositif d'application (1) pour l'application du produit pour le traitement de lésions cutanées et verrues selon l'une ou plusieurs des revendications précédentes, **caractérisé en ce qu'il** est du type en forme de stylo, c'est-à-dire, du type comprenant un corps tubulaire (2) contenant ledit produit, une pointe d'applicateur (3) fixée sur une extrémité (21) dudit corps tubulaire (2), un système de distribution (4) fixé sur l'extrémité opposée (22) dudit corps tubulaire (2).
